# EUROPÄISCHE PATENTSCHRIFT

(11) **EP 3 255 408 B1**
(45) Veröffentlichungstag und Bekanntmachung des Hinweises auf die Patenterteilung: **23.01.2019**
(21) Anmeldenummer: 17182219.0
(22) Anmeldetag: 08.05.2014
(51) Int. Cl.: G01N 15/14, G01N 15/12, G01N 15/10, C12M 1/00, G01N 33/487

(54) **DÜSE UND VERFAHREN FÜR DIE DURCHFLUSSZYTOMETRIE**
NOZZLE AND METHOD FOR FLOW CYTOMETRY
BUSE ET PROCÉDÉ POUR LA CYTOMÉTRIE DE FLUX

(30) Priorität: 08.05.2013 DE 102013208584; 17.05.2013 EP 13168370
(43) Veröffentlichungstag der Anmeldung: 13.12.2017
(62) Teilanmeldung aus: 14724060.0
(73) Patentinhaber: Masterrind GmbH, 27283 Verden (DE)
(72) Erfinder: RATH, Detlef, 31535 Neustadt (DE)
(74) Vertreter: Taruttis, Stefan Georg

(56) Entgegenhaltungen:
- EP-A2- 0 526 131
- EP-A2- 2 522 983
- US-A- 3 871 770
- US-A- 4 983 038
- US-A- 4 988 619

## Beschreibung

Die vorliegende Erfindung betrifft eine Düse für ein Durchflußzytometer, und ein Verfahren, das mit der Düse bzw. mit einem die Düse aufweisenden Durchflußzytometer durchführbar ist, zur Ausrichtung von Partikeln in einem Flüssigkeitsstrom, der einen partikelhaltigen Kernstrom innerhalb eines Hüllstroms aufweist oder daraus besteht. Insbesondere betrifft die Erfindung die Verwendung der Düse in einem Verfahren zur Ausrichtung von Partikeln und Sortierung der Partikel in Abhängigkeit von einer Eigenschaft, die im Anschluss an den Durchtritt der Partikel durch die Düse detektiert wird. Dabei ist die Sortierung vorzugsweise das Ablenken von Abschnitten des Flüssigkeitsstroms, insbesondere von Tropfen, die aus dem Flüssigkeitsstrom gebildet sind, in zumindest zwei Fraktionen. In dieser Ausführungsform betrifft die Erfindung die Herstellung von einer Fraktion von Partikeln durch Ausrichtung der Partikel in einem Flüssigkeitsstrom mit der erfindungsgemäßen Düse.

Bevorzugt sind die Partikel, die mit dem Verfahren ausgerichtet und optional in Fraktionen sortiert werden, von insgesamt flacher Gestalt, und sind beispielsweise zu einer Schnittebene symmetrisch, sodass die Partikel senkrecht zu ihrer Längsachse einen Querschnitt mit einer ersten und einer zweiten Dimension aufweisen, wobei der Querschnitt in der ersten Dimension kleiner ist als in der zweiten Dimension. Bevorzugte Partikel sind biologische Zellen, beispielsweise Blutzellen, insbesondere plättchenförmige Zellen und nicht-menschliche Säugetierspermien, insbesondere von einem männlichen Tier gewonnene Spermien, das insbesondere ein Rind, Schwein, Schaf, Elefant, Kamel, Pferd oder ein Ziegenbock ist.

### Stand der Technik

Die US 6,149,867 beschreibt eine gattungsgemäße Vorrichtung und ein Verfahren mit einer Düse zur Sortierung von Säugerspermien in geschlechtschromosomenspezifische Spermienfraktionen. Das Verfahren verwendet ein Durchflusszytometer mit einer Düse, deren Gehäuse konisch zuläuft und in dem eine Zuleitung für die Spermien enthaltende Kernflüssigkeit enthalten ist, sodass am Düsenauslass ein Spermien enthaltender Kernstrom von einem Hüllstrom umgeben austritt.

Die WO 99/05504 beschreibt eine Düse zur Verwendung in einem Durchflusszytometer zur Sortierung von Spermien, die sich dadurch auszeichnet, dass das Gehäuse der Düse in dem Abschnitt zwischen der Auslassöffnung eines Zuführröhrchens für Spermien enthaltende Kernstromflüssigkeit eine im Wesentlichen trichterförmig zulaufende Innenoberfläche hat, die einen ersten ellipsenförmigen Querschnitt hat, und einen daran angrenzenden axialen Abschnitt mit ellipsenförmigem Querschnitt, der um 90° zu dem ellipsenförmigen Querschnitt des stromaufwärts gelegenen Abschnitts gedreht ist.

Die EP 1238261 B1 beschreibt eine Düse für ein Durchflusszytometer zur Sortierung von Spermien, bei dem ein in einer Düse koaxial angeordnetes Zuführröhrchen für Spermien enthaltende Kernstromflüssigkeit von einem Abschnitt mit zylindrischem Außendurchmesser zu einem im Wesentlichen rechteckigen Querschnitt innerhalb eines kegelstumpfförmigen Abschnitts der Düse zuläuft, wobei sich stromabwärts dieses Zulaufröhrchens ein sich verjüngender Abschnitt der Düse mit elliptischem Querschnitt anschließt.

Die US 4,988,619 beschreibt eine Düse zur Ausrichtung von Zellen für die Sortierung, bei der in einer rohrförmigen Durchflusskammer, die sich zu einer Düse verjüngt, koaxial ein Röhrchen angeordnet ist, das vor dem sich verjüngenden Abschnitt endet, wobei sich eine Finne, die flach und massiv ist, über den Durchmesser des zylindrischen Abschnitts erstreckt und das Röhrchen mit der Wand des zylindrischen Abschnitts verbindet. Die Finne läuft in ihrer unteren Kante spitz zu.

Die US 3,871,770 beschreibt eine Düse zur hydrodynamischen Fokussierung von Blutzellen und weist um ein koaxial laufendes Röhrchen einen Stator auf, der einen rotationssymmetrischen Verdrängungskörper entlang des Röhrchens und Leitschaufeln hat, die sich vom Verdrängungskörper bis zur Wandung der das Röhrchen umgebenden Düse erstrecken und die das Röhrchen umgebende Hüllflüssigkeit in eine Rotationsbewegung versetzen sollen.

Die EP 2522983 A2 beschreibt eine Düse zur Partikelausrichtung, die sich dadurch auszeichnet, dass das Zuführungsröhrchen, das im Innenvolumen der Düse angeordnet ist, einen gestreckten Querschnitt aufweist. Ein Leitelement, das sich von dem Zuführungsröhrchen bis in einem Abstand zur Innenwand in dem sich verjüngenden Abschnitt des Gehäuses erstreckt, ist in der EP 2522983 A2 nicht gezeigt.

Die EP 0526131 A2 beschreibt eine Düse zur Erzeugung eines flachen Probenstroms innerhalb eines Hüllstroms. Die EP 2522983 A2 zeigt ähnlich der US 4,988,619 einen Strömungsteiler, der sich im zylindrischen Teil des umfassenden Gehäuses von dem zentralen Zuführungsröhrchen bis bündig an die Innenwand des Gehäuses erstreckt. Ein Leitelement ist nicht in dem sich verjüngenden Abschnitt der Düse angeordnet, und erstreckt sich auch nicht nur bis in einem Abstand von der Innenwand des Gehäuses.

Die US 2004/0050186 A1 betrifft eine Düse, bei der die Ausrichtung von Partikeln durch eine zulaufende Düse mit elliptischem Innenquerschnitt erreicht wird.

### Aufgabe der Erfindung

Gegenüber dem Stand der Technik liegt die Aufgabe der Erfindung darin, eine alternative Düse zur Ausrichtung von Partikeln in einem Flüssigkeitsstrom und ein Verfahren zur Ausrichtung nicht rotationssymmetrischer Partikel in einem Flüssigkeitsstrom bereitzustellen. Eine bevorzugte Aufgabe liegt in der Bereitstellung einer Düse, die einfach herzustellen ist, insbesondere nur Bauteile mit Innenflächen aufweist, die rotationssymmetrisch sind und daher einfach zu erzeugen sind.

### Allgemeine Beschreibung der Erfindung

Die Erfindung löst die Aufgabe mit den Merkmalen der Ansprüche und betrifft eine Düse, deren Gehäuse sich zu einem Auslass verjüngt und in dem ein Zuführungsrohr für eine Kernstromflüssigkeit angeordnet ist, dessen Austrittsöffnung in einem Abstand vor dem Auslass des Gehäuses angeordnet ist. Der Auslass des Gehäuses bildet den Auslass der Düse. Das Gehäuse der Düse erstreckt sich von seinem Auslass, der an dessen ersten Ende angeordnet ist, zu seinem gegenüberliegenden zweiten Ende und weist einen mit dem Innenvolumen verbundenen Einlass für eine Hüllstromflüssigkeit auf. Das zweite Ende des Gehäuses kann mit einem Deckel überdeckt sein, in dem optional der Einlass für Hüllstromflüssigkeit angeordnet ist. Bevorzugt ist der Innenquerschnitt des Gehäuses rotationssymmetrisch bzw. kreisförmig und wird entsprechend durch eine rotationssymmetrische Innenfläche gebildet, die zumindest abschnittsweise kegelförmig zum Auslass zuläuft. Das innerhalb des Gehäuses angeordnete Zuführungsrohr ist bevorzugt koaxial zur Längsachse des Gehäuses angeordnet. Die Eintrittsöffnung des Zuführungsrohrs ist mit einer Zuleitung für eine partikelhaltige Kernstromflüssigkeit verbunden. Das Zuführungsrohr besteht vorzugsweise aus Metall.

Die erfindungsgemäße Düse zeichnet sich dadurch aus, dass sich in dem Gehäuse beidseitig des Zuführungsrohrs ein Leitelement mit einem Querschnitt erstreckt, der sich weiter in einer ersten Dimension senkrecht zur Längsachse des Gehäuses erstreckt als in einer zur ersten Dimension senkrecht angeordneten zweiten Dimension, die auch als Dicke des Leitelements bezeichnet wird. Das Leitelement, das sich in der Düse beidseitig des Zuführungsrohrs erstreckt, begünstigt die Ausrichtung von Partikeln, die in dem Flüssigkeitsstrom, insbesondere in dem Kernstrom enthalten sind, insbesondere so, dass Partikel mit gestrecktem Querschnitt in eine gemeinsame Ausrichtung gebracht werden, bevorzugt in eine Ausrichtung, in der die längere Erstreckung des Querschnitts der Partikel etwa parallel zur ersten Dimension des Leitelements angeordnet ist. Das Zuführungsrohr kann innerhalb des Leitelements angeordnet sein oder als Bohrung innerhalb des Leitelements ausgebildet sein. Das Leitelement erstreckt sich daher in der ersten Dimension bis in einen geringeren Abstand zur Innenwand des Gehäuses, während es in der zweiten Dimension weiter von der Innenwand des Gehäuses beabstandet ist. Das Leitelement endet in einer Kante, die das Leitelement in seiner ersten Dimension begrenzt und die Dicke der zweiten Dimension aufweist. Das Leitelement bildet mit der Innenwand des Gehäuses einen lichten Querschnitt, der in zwei durch das Leitelement beabstandete Anteile geteilt ist, die sich bevorzugt ausschließlich in dem Bereich des lichten Querschnitts kontaktieren bzw. zusammenlaufen, um den das Leitelement in der ersten Dimension von der Innenwand des Gehäuses beabstandet ist. Das Leitelement weist eine Dicke auf, die sich in der zweiten Dimension seines Querschnitts erstreckt, die geringer als seine Erstreckung entlang seiner ersten Dimension ist. In der zweiten Dimension kann sich das Leitelement z.B. zu maximal 70%, bevorzugt maximal 50%, bevorzugter maximal 30%, maximal 20% oder maximal 10% im Verhältnis zur Erstreckung in der ersten Dimension erstrecken. In der zweiten Dimension kann sich der Querschnitt des Leitelements entlang der ersten Dimension verändern, insbesondere sich entlang der ersten Dimension von der Längsachse des Gehäuses bis zu seiner Kante verringern oder vergrößern.

Entlang der Längsachse des Gehäuses kann sich das Leitelement in seiner ersten Dimension bis in den gleichen Abstand von der Längsachse des Gehäuses bzw. bis zur Innenwand des Gehäuses erstrecken, oder sich mit längs der Längsachse des Gehäuses unterschiedlichem Abstand erstrecken, bzw. bis in einen unterschiedlichen Abstand von der Innenwand des Gehäuses. Der Querschnitt des Leitelements kann daher in seiner ersten Dimension in einer Kante enden, die längs der Längsachse des Gehäuses im gleichen Abstand zur Innenwand des Gehäuses angeordnet ist, oder längs der Längsachse des Gehäuses in unterschiedlichem Abstand von der Innenwand des Gehäuses. Z.B. kann die Kante in einem an das erste Ende des Leitelements angrenzenden Abschnitt, generell auch erster Endabschnitt genannt, in geringerem Abstand von der Innenwand des Gehäuses angeordnet sein als in einem daran angrenzenden Abschnitt, der der Austrittsöffhung des Zuführungsrohrs gegenüberliegt. Die Kante kann z.B. in dem an das erste Ende des Leitelements angrenzenden Abschnitt in einem längs der Längsachse des Gehäuses gleichen Abstand zur Innenwand des Gehäuses angeordnet sein und in dem angrenzenden Abschnitt, der der Austrittsöffnung gegenüberliegt, mit einem Abstand zur Innenwand des Gehäuses, der mit zunehmendem Abstand von der Austrittsöffnung größer wird. Die Kante ist abschnittsweise bogenförmig, konvex oder konkav in Bezug zur Längsachse des Gehäuses. In Bezug zur zweiten Dimension kann die Kante plan, konkav oder konvex sein, insbesondere parallel zur Innenwand des Gehäuses.

Das Leitelement weist bevorzugt in seinem an sein erstes Ende angrenzenden ersten Endabschnitt eine zur Innenwand des Gehäuses im Wesentlichen parallele Kante auf. Der erste Endabschnitt ist insbesondere im sich verjüngenden Abschnitt der Düse angeordnet, die an die Auslassöffnung angrenzt. Diese Anordnung des ersten Endabschnitts im sich verjüngenden Abschnitt des Gehäuses erlaubt eine effektive Ausrichtung von flachen Zellen, insbesondere von nicht-menschlichen Säugerspermien. Der erste Endabschnitt des Leitelements weist mit zunehmendem Abstand vom ersten Ende des Leitelements eine größere Erstreckung in der ersten Dimension auf, so dass die Kanten des ersten Endabschnitts in einem Abstand zur Innenwand des Gehäuses im Wesentlichen parallel zum sich verjüngenden, insbesondere kegelförmig zulaufenden Gehäuseabschnitt verlaufen. Die Kanten des ersten Endabschnitts können dabei bogenförmig, insbesondere konvex, in einem Abstand zum sich verjüngenden, insbesondere kegelförmig zulaufenden Gehäuseabschnitt verlaufen. Der erste Endabschnitt erlaubt dessen Anordnung in diesem sich verjüngenden Abschnitt der Düse. Der erste Endabschnitt kann Kanten aufweisen, die in einem Abstand von 1 bis 10%, z.B. 2 bis 5% des Abstands zwischen der Längsachse des Gehäuses und/oder des Zuführungsrohrs zur Gehäusewand beträgt. Angrenzend an den ersten Endabschnitt und gegenüber seinem ersten Ende weist das Leitelement einen zweiten Endabschnitt auf, der sich in Richtung auf das zweite Ende verjüngt. Das Leitelement kann sich in seinem zweiten Endabschnitt gegenüber dem ersten Endabschnitt, bzw. an seinem zweiten Ende, so verjüngen, dass es am Zuführungsrohr ausläuft oder endet. In dieser Ausführungsform bildet das Zuführungsrohr den Träger bzw. die Verbindung zwischen dem Deckel des Gehäuses und dem Leitelement, so dass nur das Zuführungsrohr das Leitelement trägt. In dieser Ausführung bildet das Zuführungsrohr einen zylindrischen Träger für das vom Deckel beabstandete Leitelement.

Zwischen erstem und zweitem Endabschnitt weist das Leitelement bevorzugt seine größte Erstreckung in der ersten Dimension auf.

Optional weist das Leitelement entlang des ersten und zweiten Endabschnitts eine schmale bzw. scharfe Kante, bzw. seine geringste Erstreckung in der zweiten Dimension auf. Eine schmale bzw. scharfe Kante kann eine laminare Strömung zwischen Leitelement und Innenwand der Düse begünstigen.

Der zweite Endabschnitt kann sich gegenüber dem ersten Endabschnitt bis an das Zuführungsrohr verjüngen, bzw. am Zuführungsrohr auslaufen. Der zweite Endabschnitt kann in einem Abstand von zumindest 20%, bevorzugter zumindest 30%, noch bevorzugter zumindest 50% der Länge des Zuführungsrohrs innerhalb des Gehäuses, bzw. der Länge des Zuführungsrohrs zwischen einem Deckel des Gehäuses und seinem ersten Ende, vom Deckel des Gehäuses beabstandet sein. In dieser Ausführungsform trägt allein das Zuführungsrohr das Leitelement, das bevorzugt einen kreisförmigen Innen- und Außenquerschnitt hat bzw. zylindrisch ist. Diese Anordnung des Leitelements in einem Abstand zum Deckel am Zuführungsrohr ist bevorzugt.

In Alternative dazu kann sich gegenüber dem ersten Endabschnitt am zweiten Endabschnitt ein Übergangsabschnitt anschließen, dessen Außenquerschnitt zunimmt und an den ein Zylinderabschnitt angrenzt. Dabei kann sich der Zylinderabschnitt bis an den Deckel erstrecken und mit dem Gehäuse einen ringförmigen lichten Querschnitt bilden. Der Zylinderabschnitt weist bevorzugt einen größeren Außendurchmesser auf als sich der zweite Endabschnitt angrenzend an den Übergangsabschnitt in seiner ersten Dimension erstreckt, so dass der zweite Endabschnitt mit dem Übergangsabschnitt eine Einschnürung in zumindest einer von erster und zweiter Dimension bildet. Insbesondere in Ausführungsformen, in denen der erste Endabschnitt, optional und bevorzugt auch der zweite Endabschnitt, einen sehr gestreckten Querschnitt haben, z.B. wenn die Erstreckung in der zweiten Dimension 10 bis 30% oder bis maximal 20% oder bis 15% der Erstreckung in der ersten Dimension beträgt, verbindet der Übergangsabschnitt die Außenflächen des Zylinderabschnitts mit denen des Leitelements. Der Zylinderabschnitt kann z.B. einen Außendurchmesser haben, der größer als die Einschnürung ist und kleiner als die größte Erstreckung des Leitelements in seiner ersten Dimension.

Für die Erzeugung eines Flüssigkeitsstroms mit tierischen Zellen als Partikel in einer Kernstromflüssigkeit kann sich die Innenwand des Gehäuses z.B. von einem Durchmesser von ca. 4 bis 10mm bis zum Auslass verjüngen, der z.B. einen Durchmesser von 0,2 bis 1 oder bis 0,5mm haben kann. Das Leitelement kann sich z.B. entlang der Längsachse des Gehäuses über 3 bis 5mm erstrecken und in der ersten Dimension von etwa 0,5mm bis 1,5 mm am ersten Ende bis etwa 3 bis 5mm maximal, während es sich in der zweiten Dimension etwa um 0,5 bis 1mm erstreckt, jeweils beidseitig von der Längsachse.

Das Leitelement ist bevorzugt zur Längsachse des Gehäuses symmetrisch und besteht insbesondere aus zwei Anteilen, die sich mit ihrer ersten Dimension in einer gemeinsamen Ebene erstrecken, in der die Längsachse des Gehäuses angeordnet ist, wobei weiter bevorzugt der Querschnitt des Leitelements entlang der Längsachse des Gehäuses symmetrisch ausgebildet ist.

Das Leitelement bewirkt eine Ausrichtung der in der Kernstromflüssigkeit enthaltenen Partikel nach deren Form in dem Abschnitt zwischen der Austrittsöffnung des Zuführungsrohrs und dem Auslass der Düse. Diese Ausrichtung der Partikel wird gegenwärtig darauf zurückgeführt, dass die Erstreckung des Leitelements in seiner zweiten Dimension zu unterschiedlichen Strömungsgeschwindigkeiten der Hüllstromflüssigkeit führt, insbesondere über die erste Dimension des Leitelements. Es wird angenommen, dass diese unterschiedlichen Strömungsgeschwindigkeiten zu einer Ausrichtung der Partikel führen, die diese im Wesentlichen auch nach Austritt aus dem Auslass der Düse aufweisen.

Die Düse hat den Vorteil, dass die Innenwand bzw. der Querschnitt ihres Gehäuses rotationssymmetrisch sein kann und daher auf einfache Weise herstellbar ist, z.B. mittels Bohren. Entsprechend verjüngt sich das Gehäuse dadurch, dass die Innenwand einen sich auf den Auslass verringernden Querschnitt aufspannt, der insbesondere rotationssymmetrisch um die Längsachse des Gehäuses ausgebildet ist. Bevorzugt ist der von der Innenwand des Gehäuses aufgespannte Querschnitt angrenzend an den Auslass kegelförmig. Optional kann der von der Innenwand des Gehäuses aufgespannte Querschnitt über den Abschnitt, der sich zwischen der Austrittsöffhung des Zuführungsrohrs und dem Auslass des Gehäuses erstreckt, sich verjüngen, insbesondere kegelförmig zulaufen. Optional kann der von der Innenwand des Gehäuses aufgespannte Querschnitt, der sich über den Abschnitt zwischen der Austrittsöffhung des Zuführungsrohrs und dem zweiten Ende des Leitelements oder bis an das zweite Ende des Gehäuses erstreckt, insbesondere angrenzend an den Abschnitt, der sich zwischen der Austrittsöffnung des Zuführungsrohrs und dem Auslass des Gehäuses erstreckt, sich verjüngen, insbesondere kegelförmig zulaufen, oder eine andere Form aufweisen, z.B. zylindrisch sein. Auch der Auslass kann rotationssymmetrisch sein, insbesondere eine runde Bohrung. Bevorzugt weist die Düse an ihrem ersten Ende einen Einsatz aus hartem Material auf, z.B. Keramik oder Saphir, in dem eine Bohrung als Auslass gebildet ist. Die Ausbildung des lichten Querschnitts der Düse, der die Strömung der Hüllstromflüssigkeit steuert, erfolgt durch das in dem Gehäuse angeordnete Leitelement, wobei der Querschnitt durch Formung der Außenfläche des Leitelements erzeugt wird, bzw. durch die unterschiedliche Erstreckung des Leitelements in seiner ersten und zweiten Dimension, die senkrecht zur Längsachse und senkrecht zueinander stehen. Die Düse hat daher den Vorteil, dass die nicht rotationssymmetrische Oberfläche des Leitelements als Außenfläche herstellbar ist, während die Innenwand des Gehäuses als rotationssymmetrische Fläche herstellbar ist.

Das Leitelement erstreckt sich von seinem ersten Ende bis zu seinem entlang der Längsachse des Gehäuses gegenüberliegenden zweiten Ende, wobei das erste Ende angrenzend an die Austrittsöffhung des Zuführungsrohrs oder in einem geringen Abstand weiter von dem Auslass des Gehäuses angeordnet ist, als die Austrittsöffnung des Zuführungsrohrs, z.B. um bis zu 10%, bevorzugt um bis zu 5% oder 2% des Abstands der Austrittsöffhung des Zuführungsrohrs vom Auslass des Gehäuses. Bevorzugt ist das erste Ende in der Ebene angeordnet, in der die Austrittsöffnung des Zuführungsrohrs liegt. Das zweite Ende des Leitelements kann an das zweite Ende des Gehäuses angrenzen oder kann in einem Abstand vom zweiten Ende des Gehäuses angeordnet sein, z.B. in einem Abstand von 1 bis 80%, bevorzugt 10 bis 50% der Erstreckung des Gehäuses von seinem Auslass zu seinem zweiten Ende oder zum zweiten Ende des Leitelements.

Bevorzugt erstreckt sich das Leitelement in seiner ersten und zweiten Dimension senkrecht zum Zuführungsrohr und entlang der Längsachse des Gehäuses, wobei insbesondere das Zuführungsrohr koaxial zur Längsachse des Gehäuses angeordnet ist.

Bevorzugt weist die Düse an der Eintrittsöffnung des Zuführungsrohrs einen Pufferbehälter für Kernstromflüssigkeit auf, in die eine Zuleitung für partikelhaltige Kernstromflüssigkeit mündet. Ein solcher Pufferbehälter erhöht den Anteil von Partikeln, die durch das Gehäuse in eine vorbestimmte Ausrichtung angeordnet werden. Dies wird gegenwärtig darauf zurückgeführt, dass ein Pufferbehälter Strömungseinflüsse aus der Zuleitung verringert, die sich ins Zuführungsrohr fortsetzen. Weiter bevorzugt weist die Düse einen Schwingungserzeuger auf, der an einer Wandung des Pufferbehälters für Kernstromflüssigkeit befestigt ist, die der Eintrittsöffnung des Zuführungsrohrs gegenüberliegt. Der Schwingungserzeuger ist bevorzugt ein mit elektrischer Spannung beaufschlagbares Piezoelement. Besonders bevorzugt ist der Schwingungserzeuger, z.B. das Piezoelement, unter Vorspannung gegen die Wand der Pufferkammer für Kernstromflüssigkeit befestigt, z.B. mit einem zwischen dem Schwingungserzeuger und dem Innenvolumen der Pufferkammer angeordneten Deckel gegen die Wand der Pufferkammer belastet.

Bevorzugt weist die Düse an der Einlassöffnung der Düse für Hüllstromflüssigkeit einen Pufferbehälter für Hüllstromflüssigkeit auf, der optional angrenzend an den Pufferbehälter für Kernstromflüssigkeit angeordnet ist, z.B. zwischen dem Pufferbehälter für Kernstromflüssigkeit und dem zweiten Ende des Gehäuses, wobei weiter optional das Zuführungsrohr durch den Pufferbehälter für Hüllstromflüssigkeit geführt ist. Ein Pufferbehälter für Hüllstromflüssigkeit verringert Einflüsse der Zuleitung von Hüllstromflüssigkeit auf die Strömung der Hüllstromflüssigkeit im lichten Querschnitt des Gehäuses und erhöht die Ausrichtung von Partikeln in eine vorbestimmte Orientierung.

Das Zuführungsrohr hat bevorzugt einen kreisförmigen Innenquerschnitt, der sich entlang der Längsachse des Gehäuses verjüngen kann und bevorzugt entlang der Längsachse des Gehäuses konstant ist.

Das Piezoelement dient als Schwingungserzeuger, der bevorzugt senkrecht zur Längsachse des Gehäuses verlaufende Druckwellen erzeugt, um bei Anordnung des Auslasses des Gehäuses in einem gasgefüllten Raum einen Tropfenstrom zu erzeugen.

Ein Durchflusszytometer mit der erfindungsgemäßen Düse weist bevorzugt zumindest eine erste Strahlungsquelle auf, die auf einen ersten Abschnitt des aus dem Gehäuse der Düse austretenden Flüssigkeitsstroms gerichtet ist, z.B. einen Laser, und einen gegenüber der Strahlungsquelle auf den ersten Abschnitt des Flüssigkeitsstroms gerichteten ersten Detektor, wobei optional der Detektor ein Signal erzeugt, das eine Auslenkungseinrichtung ansteuert, um Abschnitte des Flüssigkeitsstroms in Abhängigkeit von der Detektion mittels des Signals abzulenken, z.B. zu fraktionieren. Optional weist die Vorrichtung eine zweite Strahlungsquelle auf, die z.B. auf einen zweiten Abschnitt des Flüssigkeitsstroms zwischen der ersten Strahlungsquelle und der Düse gerichtet ist, und einen zweiten Detektor, der auf diesen zweiten Abschnitt gerichtet ist. Bevorzugt erzeugt der zweite Detektor ein zweites Signal, das die Auslenkungseinrichtung steuert, so dass Abschnitte des Flüssigkeitsstroms zusätzlich in Abhängigkeit von dem zweiten Signal ausgelenkt werden.

Die Auslenkungseinrichtung kann ein Paar elektrisch entgegengesetzt geladener Platten sein, die beidseitig des Flüssigkeitsstroms angeordnet sind, und einen elektrischen Kontakt aufweisen, der in der Düse angeordnet ist, insbesondere in dem Gehäuse. Optional kann der Kontakt das Zuführungsrohr für Kernstromflüssigkeit sein. Vorzugsweise ist der elektrische Kontakt in Abhängigkeit vom ersten und/oder zweiten Signal gesteuert, so dass eine positive oder negative Aufladung in Abhängigkeit vom ersten und/oder zweiten Signal erfolgt. Alternativ kann die Auslenkungseinrichtung ein auf den Flüssigkeitsstrom gerichteter Laser sein, der auf den Flüssigkeitsstrom gerichtet ist und eingerichtet ist, den Flüssigkeitsstrom nur oberflächlich bis zur oberflächlichen Verdampfung des Flüssigkeitsstroms zu verdampfen, wie dies z.B. in der WO2010/149739 beschrieben ist.

Das erfindungsgemäße Verfahren unter Verwendung der Düse bzw. eines Durchflusszytometers mit der Düse weist die folgenden Schritte auf:
- Bereitstellen einer partikelhaltigen Kernstromflüssigkeit,
- Bereitstellen einer Hüllstromflüssigkeit,
- Pumpen der Hüllstromflüssigkeit durch die Einlassöffnung für Hüllstromflüssigkeit,
- Pumpen der partikelhaltigen Kernstromflüssigkeit durch die Eintrittsöffnung des Zuführungsrohrs, wobei sich entlang der Längsachse des Gehäuses ein Leitelement erstreckt, wobei bevorzugt das Zuführungsrohr koaxial zur Längsachse des Gehäuses angeordnet ist, wobei das Leitelement den lichten Querschnitt des Innenvolumens des Gehäuses in zwei Teile unterteilt, und wobei das Leitelement sich entlang der Längsachse in größerem Maß entlang einer zur Längsachse senkrechten ersten Dimension bis in einen Abstand zur Innenwand des Gehäuses erstreckt als es sich in einer zur Längsachse und zur ersten Dimension senkrechten zweiten Dimension erstreckt,
- Durchströmenlassen des lichten Querschnitts des Gehäuses von Hüllstromflüssigkeit und Durchströmenlassen des Zuführungsrohrs von Kernstromflüssigkeit, wobei die Kernstromflüssigkeit nach Austritt aus der Austrittsöffnung des Zuführungsrohrs von der Hüllstromflüssigkeit kontaktiert wird und die in der Kernstromflüssigkeit enthaltenen Partikel in eine vorbestimmte Ausrichtung bewegt werden,
- optional Durchströmenlassen eines sich verjüngenden Abschnitts zwischen der Austrittsöffhung des Zuführungsrohrs und dem Auslass des Gehäuses, wodurch der Querschnitt von Hüllstromflüssigkeit und Kernstromflüssigkeit verringert wird, und
- Austretenlassen der von einer Hüllstromflüssigkeit umgebenen Kernstromflüssigkeit durch den Auslass des Gehäuses.
- Optional enthält das Verfahren den Schritt des Detektierens einer Eigenschaft der Partikel und,
- weiter optional, den Schritt des Behandelns (z.B. durch Laserbestrahlung) und/oder des Ablenkens der Partikel in getrennte Fraktionen bzw. Behälter in Abhängigkeit von einer detektierten Eigenschaft.
- Bevorzugt ist das Gehäuse rotationssymmetrisch.

Insbesondere bevorzugt ist ein Verfahren zur Herstellung geschlechtschromosomenspezifisch sortierter Fraktionen nicht-menschlicher Spermien, bei dem die Spermien durch die Düse in eine vorbestimmte Ausrichtung bewegt werden und in dieser Ausrichtung vor dem Strahlengang eines Detektors bewegt und detektiert werden.

Optional ist das Leitelement auf das Zuführungsrohr aufgeschoben, bevorzugt reversibel bzw. lösbar aufgeschoben, und z.B. am zweiten Ende des Gehäuses festgelegt. Bevorzugt ist das Leitelement einstückig mit dem Zuführungsrohr ausgebildet und an einem Ende gegenüber seiner Austrittsöffnung befestigt, z.B. mittels Eingriffs mit einem Deckel, der das Innenvolumen des Gehäuses an dessen zweitem Ende begrenzt.

Das Zuführungsrohr kann einen kreisförmigen Innenquerschnitt aufweisen. Alternativ kann das Zuführungsrohr einen gestreckten Innenquerschnitt aufweisen, z.B. einen elliptischen oder rechteckigen Innenquerschnitt, z.B. mit gerundeten Innenkanten, wobei die längere Erstreckung eines gestreckten Innenquerschnitts bevorzugt etwa parallel zur ersten Dimension angeordnet ist. Bevorzugt weist das Leitelement eine Symmetrieebene auf, durch die die Längsachse geht, und besonders bevorzugt erstreckt sich der Innenquerschnitt des Zuführungsrohrs in einer mit dem Leitelement gemeinsamen Symmetrieebene. Ein gestreckter Innenquerschnitt des Zuführungsrohrs kann z.B. ein Verhältnis der langen zur kurzen Erstreckung von maximal 0,3 oder maximal 0,2 aufweisen.

Die Düse, bevorzugt mit einem Einsatz aus Keramik oder Saphir, der den Auslass am ersten Ende des Gehäuses bildet, kann aus Kunststoff bestehen, z.B. aus PEEK oder POM, optional aus Keramik. Das Zuführungsrohr kann aus Metall bestehen und das Leitelement aus Kunststoff, z.B. aus PEEK oder POM; bevorzugt ist das Zuführungsrohr als Bohrung runden Querschnitts in dem Leitelement ausgebildet, das aus Kunststoff besteht, z.B. aus PEEK oder POM. Bevorzugt ist das Verfahren ein Verfahren zur Herstellung von Fraktionen nichtmenschlicher Säugetierspermien und weist den Schritt auf, nach dem Durchströmen des Zuführungsrohrs der Düse mit einer Kernstromflüssigkeit, die nichtmenschliche Spermien eines Individuums enthält, das Zuführungsrohr und das Leitelement gegen ein anders Zuführungsrohr und Leitelement zu tauschen oder zu sterilisieren, bevor Kernstromflüssigkeit, die nichtmenschliche Spermien eines anderen Individuums enthält, durch das Zuführungsrohr der Düse strömen gelassen wird.

Die Erfindung wird nun genauer anhand von Beispielen und mit Bezug auf die Figuren beschrieben, die schematisch in
- Figur 1 eine erfindungsgemäße Düse im Querschnitt längs der Längsachse der Düse,
- Figur 2 das in der Düse angeordnete Leitelement im Querschnitt senkrecht zur Längsachse der Düse,
- Figur 3 eine erfindungsgemäße Düse ohne deren Deckel und
- Figur 4 A)-D) Leitelemente zeigen.

In den Figuren bezeichnen gleiche Bezugsziffern funktionsgleiche Elemente.

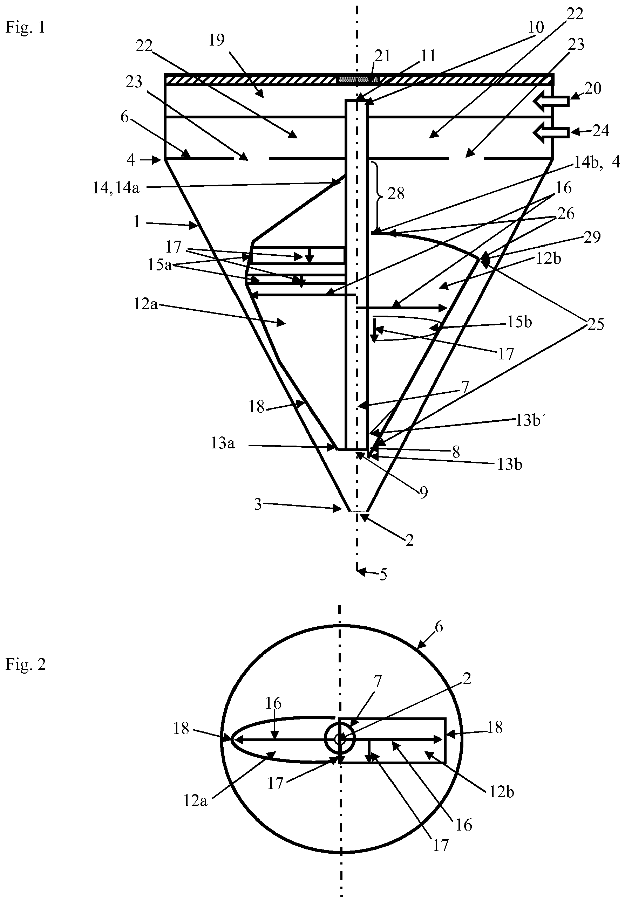

Figur 1 zeigt eine Düse mit einem Gehäuse 1, das sich von einem runden Auslass 2 an seinem ersten Ende 3 zu seinem gegenüberliegenden zweiten Ende 4 entlang einer Längsachse 5 erstreckt. Das zweite Ende 4 ist von einem Deckel 6 überdeckt, durch den sich das Zuführungsrohr 7 koaxial zur Längsachse 5 erstreckt. Das Zuführungsrohr 7 mündet an seinem ersten Ende 8 in einer Austrittsöffnung 9 und weist an seinem gegenüberliegenden zweiten Ende 10 eine Eintrittsöffnung 11 für eine partikelhaltige Kernstromflüssigkeit auf. Das Leitelement 12a, 12b ist in einem Abstand zum Deckel 6, der das Gehäuse 1 an dessen erstem Ende 4 abschließt, angeordnet und wird vom Zuführungsrohr 7 getragen.

Figur 1 zeigt das Leitelement 12a, 12b in verschiedenen Gestaltungen, die jeweils hälftig beidseits des Zuführungsrohrs 7 dargestellt sind, so dass sich eine Gestaltung jeweils symmetrisch zur Längsachse 5 erstreckt. Das Leitelement 12a, 12b erstreckt sich von seinem ersten Ende 13a, 13b, das vom ersten Ende 8 des Zuführungsrohrs 7 beabstandet ist, zu seinem zweiten Ende 14a, 14b. Am Beispiel des Leitelements 12b ist entsprechend der bevorzugten Ausführungsform gezeigt, dass sich dessen erstes Ende 13b am ersten Ende 8 des Zuführungsrohrs 7 befinden kann und bündig mit diesem abschließen kann. Das zweite Ende 14b des alternativen Leitelements 12b zeigt, dass sich ein Leitelement 12a, 12b in unterschiedlichem Ausmaß entlang der Längsachse 5 des Gehäuses 1 erstrecken kann, z.B. in einem Abstand weiter vom Auslass des Gehäuses 1 angeordnet sein kann als die Austrittsöffhung 9 oder dass das Leitelement 12b das Zuführungsrohr 7 überragen kann, wie am ersten Ende 13b gezeigt, oder z.B. dass dessen erstes Ende 13b' um einen geringen Abstand weiter vom Auslass des Gehäuses angeordnet ist. Die schematisch eingezeichneten Querschnitte 15a, 15b des Leitelements 12a, 12b erstrecken sich senkrecht zur Längsachse 5 in einer ersten Dimension 16, zu der sich die Dicke des Leitelements 12a, 12b in der dazu senkrechten zweiten Dimension 17 erstreckt. Wie an Querschnitt 15a beispielhaft gezeigt ist, kann das Leitelement 12a eine konstante Dicke in der zweiten Dimension 17 aufweisen, oder wie beispielhaft am Querschnitt 15b gezeigt ist, kann das Leitelement 12b einen Querschnitt 15b aufweisen, der sich in der zweiten Dimension 17 entlang der ersten Dimension 16 verändert, z.B. mit zunehmendem Abstand zur Längsachse 5 verringert.

In Figur 1 ist gezeigt, dass sich das Leitelement 12a, 12b insbesondere in einem Abschnitt, der an sein erstes Ende 13a, 13b angrenzt, entlang der ersten Dimension 16 von der Längsachse 5 bis in einen etwa konstanten Abstand von der Innenfläche des Gehäuses 1 erstreckt, bzw. sich bis zu einer Kante 18 in einem etwa konstanten Abstand von der Innenfläche des Gehäuses 1 erstreckt.

Der an das erste Ende 13a, 13b des Leitelements 12a, 12b angrenzende erste Endabschnitt 25 des Leitelements 12a, 12b ist innerhalb des kegelförmig auf den Auslass 2 zulaufenden Abschnitts des Gehäuses 1 angeordnet. Der zweite Endabschnitt 26 grenzt gegenüber dem ersten Ende 13a, 13b des Leitelements 12a, 12b an den ersten Endabschnitt 25 an, wobei das Leitelement dort, wo der erste und zweite Endabschnitt 25, 26 aneinander angrenzen, seine größte Erstreckung 29 in der ersten Dimension aufweist. Die Ausgestaltung des Leitelements 12a, 12b so, dass dessen erster Endabschnitt 25 innerhalb des kegelförmig zulaufenden Abschnitts des Gehäuses 1 angeordnet ist, erlaubt eine effektive Ausrichtung von Zellen mit flacher Gestalt, z.B. von nicht-menschlichen Säugerspermien, für deren anschließende Sortierung.

Figur 1 zeigt, dass die Kante 18 des ersten Endabschnitts 25 im Wesentlichen parallel und in einem Abstand zur Innenwand des Gehäuses 1 verläuft und dabei leicht konvex ausgebildet sein kann. Generell kann in dieser Ausführungsform das Leitelement 12a, 12b auf das Zuführungsrohr 7 aufgesteckt sein, optional geklemmt oder mittels eines Rastverschlusses oder eines Gewindes am Zuführungsrohr 7 angeordnet.

Entsprechend der bevorzugten Ausführungsform zeigt Figur 1 eine Düse mit einem Pufferbehälter 19 für Kernstromflüssigkeit, in dem eine Zuführleitung 20 für Kernstromflüssigkeit mündet und an dem die Eintrittsöffnung 11 des Zuführungsrohrs 7 angeschlossen ist. Ein Piezoelement 21 ist unter Vorspannung in dem Pufferbehälter 19 für Kernstromflüssigkeit gegenüber der Eintrittsöffnung 11 des Zuführungsrohrs 7 befestigt. Weiter ist ein Pufferbehälter 22 für Hüllstromflüssigkeit mit dem Einlass 23 für Hüllstromflüssigkeit verbunden, die in das Gehäuse 1 münden, wobei der Pufferbehälter 22 für Hüllstromflüssigkeit mit einer Zuführleitung 24 für Hüllstromflüssigkeit verbunden ist. Der Pufferbehälter 22 für Hüllstromflüssigkeit ist zwischen dem zweiten Ende 4 der Düse 1 und dem Pufferbehälter 19 für Kernstromflüssigkeit angeordnet, wobei das Zuführungsrohr 7 durch den Pufferbehälter 22 für Hüllstromflüssigkeit angeordnet ist.

Figur 2 zeigt senkrecht zur Längsachse 5 der Düse bzw. des Gehäuses 1 liegende Querschnitte 15a, 15b durch das Leitelement 12a, 12b, wobei nur eine Hälfte des Leitelements 12a, 12b gezeigt ist, das sich spiegelsymmetrisch zur zweiten Dimension 17 bzw. zur Längsachse 5 erstreckt. Bevorzugt ist das Zuführungsrohr 7 kreisförmig um die Längsachse 5 des Gehäuses 1 angeordnet, die im Schnittpunkt der ersten Dimension 16 und der zweiten Dimension 17 liegt. Wie generell bevorzugt, ist der Auslass 2 des Gehäuses 1 symmetrisch um die Längsachse 5 des Gehäuses 1 angeordnet.

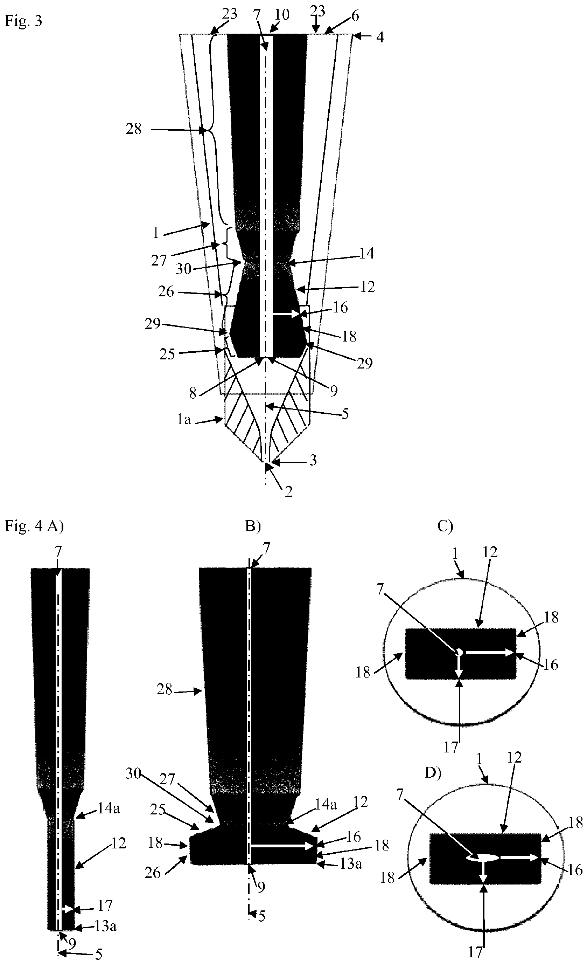

Figur 3 zeigt eine Düse, in deren Gehäuse 1 ein Leitelement 12a angeordnet ist, in dem das Zuführungsrohr 7 als Bohrung ausgeführt ist, die koaxial zur Längsachse 5 des Gehäuses 1 angeordnet ist. Der Auslass 2 des Gehäuses 1 ist durch einen Einsatz 1a gebildet, der in einem an den Auslass 2 angrenzenden Abschnitt kegelstumpfförmig ist und daran angrenzend zylindrisch. Der erste Endabschnitt 25 ist, wie generell bevorzugt, innerhalb des sich verjüngenden Abschnitts des Gehäuses 1 angeordnet. Das Leitelement 12a, 12b weist angrenzend an seinen zweiten Endabschnitt 26 einen Übergangsabschnitt 27 auf, an den wiederum ein Zylinderabschnitt 28 angrenzt. Der Zylinderabschnitt 28 erstreckt sich über die gesamte Länge des Zuführungsrohrs 7. Der Übergangsabschnitt 27 bildet mit dem zweiten Endabschnitt 26 des Leitelements 12 eine Einschnürung 30.

Figur 4A) zeigt das Leitelement 12a von Figur 3 in verkleinerter Darstellung um 90° um die Längsachse 5 gedreht. Aus Figur 3 und 4A) wird deutlich, dass sich das Leitelement 12a in der zur Längsachse 5 senkrechten ersten Dimension 16 weiter erstreckt, als in der zur Längsachse 5 und zur ersten Dimension 16 senkrechten zweiten Dimension 17.

Figur 4B) zeigt eine optionale Ausführung eines Leitelements 12a, das sich in einem an die Austrittsöffhung 9 angrenzenden Abschnitt wesentlich weiter in seiner ersten Dimension 16 erstreckt als in seiner zweiten Dimension 17, die senkrecht zur Bildebene liegt, während der Querschnitt des Leitelements 12a in einem angrenzenden Abschnitt, der von der Austrittsöffhung 9 beabstandet ist, in der ersten Dimension 16 verjüngt ist.

Figur 4C) zeigt einen zur Längsachse 5 des Gehäuses senkrechten Querschnitt 15a eines Leitelements 12a, in dem das Zuführungsrohr 7 als zentrale Bohrung mit rundem Querschnitt ausgebildet ist.

Figur 4D) zeigt ein Leitelement entsprechend Figur 4C), bei dem das Zuführungsrohr 7 einen elliptischen Innenquerschnitt aufweist, dessen lange Erstreckung parallel zu der ersten Dimension 16 liegt und dessen kurze Erstreckung parallel zur zweiten Dimension 17 liegt. Überdies zeigt Figur 4D), dass der Innenquerschnitt des Zuführungsrohrs 7 dieselbe Symmetrieebene aufweist, die durch die Längsachse (senkrecht zur Darstellungsebene) geht, wie der Querschnitt des Leitelements 12a.

### Beispiel 1: Detektion Y-Chromosomen-haltiger Spermien in Frischsamen und geschlechtsspezifische Sortierung

Frisch gewonnener Bullensamen wurde in üblicher Weise in Verdünner verdünnt und mit DNA-spezifischem Farbstoff, z.B. Bisbenzimid H 33342 (Hoechst), für 30 bis 60 min bei einer Temperatur von 20 °C bis 40 °C inkubiert und anschließend in einem Durchflusszytometer gemäß US 5125759 oder der DE 10 2005 044 530 mit Licht der Anregungswellenlänge für den Farbstoff bestrahlt. Die jeweilige Emission wurde gemessen. Die Ausrichtung der Spermien wurde mit einem Detektor bestimmt, der unmittelbar stromabwärts der Düse auf den austretenden Flüssigkeitsstrom aus vereinzelten Tropfen gerichtet war. Der Gesamt-DNA-Gehalt wurde mit einem weiteren Detektor bestimmt, der weiter stromabwärts auf den Flüssigkeitsstrom gerichtet war. Die Ablenkungseinrichtung wies zwei entgegengesetzt geladene Platten beidseitig des Flüssigkeitsstroms auf und einen Kontakt zur elektrischen Aufladung der Flüssigkeit in der Düse. Diese Aufladung wurde wie bekannt in Abhängigkeit von dem Signal des Detektors aufgegeben, der die Ausrichtung der Spermien bestimmt, und die Polarität der Aufladung in Abhängigkeit von dem Signal des Detektors zur Bestimmung des Gesamt-DNA-Gehalts. Auf diese Weise wurden die Spermatozoen abhängig von dem detektierten Signal durch ein elektrisches Feld in geschlechtschromosomspezifische Fraktionen abgelenkt.

Optional wurde ein Fluorid zur Immobilisierung der Spermien zugesetzt, z.B. in die während des Sortierverfahrens verwendete Hüll- oder Transportflüssigkeit, und/oder vor oder während des Zusatzes des Farbstoffs, um die Penetration des Farbstoffs in die Spermatozoen zu erhöhen. Fluoridionen wurden im Bereich von 0,1 bis 100 mM, vorzugsweise von 10 nM bis 10 mM zugesetzt. Es wurde gefunden, dass die optimale Konzentration des Fluorids, z.B. NaF oder KF, zwischen verschiedenen Spezies und für Individuen abwich. Die optimale Konzentrationen für die Spezies ist spezifisch und konnte allgemein als die Konzentration bestimmt werden, die bei mikroskopischer Betrachtung eine Immobilisierung von wenigstens 90% der Spermatozoen ergab, vorzugsweise von im wesentlichen allen Spermatozoen. Entsprechend bezieht sich die vorliegende Erfindung auch auf Zusammensetzungen der mit dem erfindungsgemäßen Verfahren hergestellten Spermafraktionen, und auf Verfahren zur Herstellung von geschlechtsspezifischen Spermafraktionen und anschließenden Konservierung der Spermafraktionen von nicht-menschlichen Säugetieren, jeweils vorzugsweise in Anwesenheit von Fluorid und/oder Antioxidationsmitteln.

### Bezugszeichenliste:

- 1: Gehäuse
- 1a: Einsatz
- 2: Auslass
- 3: erstes Ende des Gehäuses
- 4: zweites Ende des Gehäuses
- 5: Längsachse
- 6: Deckel
- 7: Zuführungsrohr
- 8: erstes Ende des Zuführungsrohrs
- 9: Austrittsöffhung
- 10: zweites Ende des Zuführungsrohrs
- 11: Eintrittsöffnung
- 12a, 12b: Leitelement
- 13a, 13b, 13b': erstes Ende des Leitelements
- 14, 14a, 14b: zweites Ende des Leitelements
- 15a, 15b: Querschnitt des Leitelements
- 16: erste Dimension
- 17: zweite Dimension
- 18: Kante
- 19: Pufferbehälter für Kernstromflüssigkeit
- 20: Zuführleitung für Kernstromflüssigkeit
- 21: Piezoelement
- 22: Pufferbehälter für Hüllstromflüssigkeit
- 23: Einlass für Hüllstromflüssigkeit
- 24: Zuführleitung für Hüllstromflüssigkeit
- 25: erster Endabschnitt
- 26: zweiter Endabschnitt
- 27: Übergangsabschnitt
- 28: Zylinderabschnitt
- 29: größte Erstreckung in erster Dimension
- 30: Einschnürung

## Patentansprüche

1. Düse für ein Durchflusszytometer mit einem Gehäuse (1), das eine zu seiner Längsachse (5) rotationssymmetrische Innenwand aufweist und dessen Innenquerschnitt sich entlang seiner Längsachse (5) von seinem zweiten Ende (4) zu einem Auslass (2) an seinem ersten Ende (3) verjüngt und in dem für eine Kernstromflüssigkeit ein Zuführungsrohr (7) mit kreisförmigem oder gestrecktem Innenquerschnitt angeordnet ist, das eine Eintrittsöffnung (11) aufweist und das in einer an seinem ersten Ende (8) angeordneten Austrittsöffhung (9) in einem Abstand zum Auslass (2) mündet, wobei ein Einlass (23) für eine Hüllstromflüssigkeit an dem Gehäuse (1) angeordnet ist,
mit
einem Leitelement (12), das sich entlang der Längsachse (5) in größerem Maß entlang einer zur Längsachse (5) senkrechten ersten Dimension (16) bis in einen Abstand zur Innenwand des Gehäuses (1) erstreckt als es sich in einer zur Längsachse (5) und zur ersten Dimension (16) senkrechten zweiten Dimension (17) erstreckt, wobei sich das Leitelement (12) von seinem ersten Ende (13), das in der Ebene des ersten Endes (8) des Zuführungsrohrs (7) liegt oder um einen Abstand gegenüber vom ersten Ende (8) des Zuführungsrohrs versetzt ist, bis zu seinem zweiten Ende (14, 14a, 14b) erstreckt, das in einem Abstand vom zweiten Ende (4) der Düse (1) angeordnet ist, **dadurch gekennzeichnet, dass** das Leitelement (12) an seinem ersten Ende (13) einen ersten Endabschnitt (25) aufweist, der in einem sich verjüngenden Abschnitt des Gehäuses (1) angeordnet ist und dessen Kanten in einem Abstand zur Innenwand des Gehäuses (1) verlaufen und dass das Leitelement (12) einen gegenüber dem ersten Ende (13) daran angrenzenden zweiten Endabschnitt (26) aufweist, der sich in Richtung auf das dem ersten Ende (13) gegenüberliegende zweite Ende (14, 14a, 14b) des Leitelements (12) in seiner ersten Dimension verjüngt, wobei das Leitelement (12) zwischen erstem und zweiten Endabschnitt (25, 26) seine größte Erstreckung (29) in der ersten Dimension (16) hat und das Leitelement (12) eine Dicke aufweist, die sich in der zweiten Dimension (17) seines Querschnitts erstreckt, die geringer ist als seine Erstreckung in der ersten Dimension (16), wobei die Kanten des ersten Endabschnitts bogenförmig, insbesondere konvex, in einem Abstand zum sich verjüngenden, zulaufenden Gehäuseabschnitt verlaufen.

2. Düse nach Anspruch 1, **dadurch gekennzeichnet, dass** das Leitelement (12) durch das Zuführungsrohr (7) getragen wird, dass das Leitelement an seinem zweiten Ende (14, 14a, 14b) auf dem Zuführungsrohr (7) ausläuft und dass der Abstand des zweiten Endes (14, 14a, 14b) des Leitelements (12) zumindest 20% der Länge des Zuführungsrohrs (7) beträgt, der innerhalb des Gehäuses (1) angeordnet ist.

3. Düse nach einem der Ansprüche 1 bis 2, **dadurch gekennzeichnet, dass** angrenzend an das zweite Ende (14, 14a, 14b) des Leitelements (12) ein Übergangsabschnitt (27) angeordnet ist, der mit dem Leitelement (12) eine Einschnürung (30) bildet und gegenüber dem Leitelement (12) und angrenzend an den Übergangsabschnitt (27) ein Zylinderabschnitt (28) angrenzt, der sich entlang des Zuführungsrohrs (7) erstreckt.

4. Düse nach einem der voranstehenden Ansprüche, **dadurch gekennzeichnet, dass** das erste Ende (13a) des Leitelements (12a) in der Ebene angeordnet ist, in der sich die Austrittsöffhung (9) erstreckt.

5. Düse nach einem der voranstehenden Ansprüche, **dadurch gekennzeichnet, dass** sich die zweite Dimension (17) des Querschnitts (15a, 15b) des Leitelements (12, 12a, 12b) mit zunehmendem Abstand von der Längsachse (5) verringert.

6. Düse nach einem der voranstehenden Ansprüche, **dadurch gekennzeichnet, dass** das Leitelement (12) sich in einem an sein erstes Ende (13) angrenzenden ersten Endabschnitt (25) in der ersten Dimension (16) bis zu einer Kante (18) erstreckt, die parallel zur Innenwand des Gehäuses (1) verläuft.

7. Düse nach einem der voranstehenden Ansprüche, **dadurch gekennzeichnet, dass** sich das Zuführungsrohr (7) koaxial zur Längsachse (5) erstreckt.

8. Düse nach einem der voranstehenden Ansprüche, **dadurch gekennzeichnet, dass** das Zuführungsrohr (7) in dem Leitelement (12, 12a, 12b) als Bohrung ausgebildet ist.

9. Düse nach einem der voranstehenden Ansprüche, **dadurch gekennzeichnet, dass** das Zuführungsrohr (7) in dem Leitelement (12, 12a, 12b) befestigt ist.

10. Düse nach einem der voranstehenden Ansprüche, **dadurch gekennzeichnet, dass** das Leitelement (12, 12a, 12b) mit dem Zuführungsrohr (7) an einem Deckel (6) reversibel festlegbar ist, der das Gehäuse (1) an seinem zweiten Ende (4) überdeckt.

11. Düse nach einem der voranstehenden Ansprüche, **dadurch gekennzeichnet, dass** sie einen Pufferbehälter (19) für Kernstromflüssigkeit aufweist, in den die Eintrittsöffnung (11) des Zuführungsrohrs (7) mündet.

12. Düse nach einem der voranstehenden Ansprüche, **dadurch gekennzeichnet, dass** sie einen Pufferbehälter (22) für Hüllstromflüssigkeit aufweist, der mit dem Einlass (23) für Hüllstromflüssigkeit verbunden ist.

13. Düse nach einem der voranstehenden Ansprüche, **dadurch gekennzeichnet, dass** die Kanten des ersten Endabschnitts in einem sich verjüngenden, kegelförmig zulaufenden Gehäuseabschnitt verlaufen.

14. Düse nach nach einem der voranstehenden Ansprüche, **dadurch gekennzeichnet, dass** sich das Leitelement (12) in der zweiten Dimension (17) zu maximal 50 % im Verhältnis zur Erstreckung in der ersten Dimension (16) erstreckt.

15. Düse nach einem der voranstehenden Ansprüche, **dadurch gekennzeichnet, dass** sich der Querschnitt des Leitelements (12) in der zweiten Dimension (17) entlang der ersten Dimension (16) von der Längsachse (5) des Gehäuses (1) bis zu seiner Kante (18) verringert.

16. Verfahren zur Erzeugung eines Flüssigkeitsstroms mit einem von einem Hüllstrom umgebenen partikelhaltigen Kernstrom durch Eintretenlassen einer partikelhaltigen Kernstromflüssigkeit in eine Eintrittsöffnung (11) für Kernstromflüssigkeit und Eintretenlassen einer Hüllstromflüssigkeit in eine Einlassöffnung (23) für Hüllstromflüssigkeit einer Düse nach einem der voranstehenden Ansprüche.

17. Verfahren nach Anspruch 16, **dadurch gekennzeichnet, dass** die Partikel einen Querschnitt aufweisen, der in einer zur Längsachse (5) des Gehäuses (1) der Düse senkrechten ersten Dimension (16) kleiner ist als in einer zur Längsachse (5) und zur ersten Dimension (16) senkrechten zweiten Dimension (17).

18. Verfahren nach einem der Ansprüche 16 bis 17, **dadurch gekennzeichnet, dass** in dem aus der Düse austretenden Flüssigkeitsstrom zumindest eine Eigenschaft der in dem Kernstrom enthaltenen Partikel detektiert wird und die Partikel abhängig von der detektierten Eigenschaft in zumindest zwei Fraktionen getrennt werden, oder in Abhängigkeit von der detektierten Eigenschaft unterschiedlich behandelt werden.

19. Verfahren nach einem der Ansprüche 16 bis 18 zur Herstellung eines Präparats von Partikeln, die einen Querschnitt mit einer ersten Dimension und einer zweiten Dimension aufweisen, wobei die Partikel in der ersten Dimension kleiner sind als in der zweiten Dimension, **gekennzeichnet dadurch, dass** die Partikel nicht-menschliche Säugetierspermien sind, die detektierte Eigenschaft das Vorliegen des X-Chromosoms oder des Y-Chromosoms in dem Spermium ist, und durch das Bestrahlen der Spermien in Abhängigkeit von der detektierten Eigenschaft oder das Ablenken der Spermien in Abhängigkeit von der detektierten Eigenschaft in zumindest zwei unterschiedliche Fraktionen.

## Claims

1. Nozzle for a flow cytometer having a housing (1) which has an inner wall that is rotationally symmetric to its longitudinal axis (5) and the housing having an internal cross-section that tapers along its longitudinal axis (5) from its second end (4) to an outlet (2) at its first end (3), and in which, for a core flow liquid, a feed tube (7) having a circular or a stretched internal cross-section is arranged, which feed tube (7) has an inlet opening (11) and which discharges into an outlet opening (9) arranged at its first end (8) at a distance from the outlet (2), wherein an inlet (23) for a sheath flow liquid is arranged at the housing (1),
having
a guiding element (12), which extends along the longitudinal axis (5) to a larger degree along a first dimension (16) perpendicular to the longitudinal axis (5) up to a distance to the inner wall of the housing (1) than it extends in a second dimension (17) that is perpendicular to the longitudinal axis (5) and to the first dimension (16), wherein the guiding element (12) extends from its first end (13), which lies in the plane of the first end (8) of the feed tube (7) or is offset by a distance from the first end (8) of the feed tube, up to its second end (14, 14a, 14b), which is arranged at a distance from the second end (4) of the nozzle (1), **characterized in that** the guiding element (12) at its first end (13) has a first end section (25), which is arranged in a tapered section of the housing (1) and the edges of which run at a distance from the inner wall of the housing (1), and **in that** the guiding element (12) has a second end section (26) opposite the first end (13) and adjacent to it, which is tapered towards the second end (14, 14a, 14b) of the guiding element (12) opposite the first end (13) in its first dimension, wherein the guiding element (12) has its largest extension (29) in the first dimension (16) between the first and the second end section (25, 26) and the guiding element (12) has a thickness which extends in the second dimension (17) of its cross-section which is smaller than its extension in the first dimension (16), wherein the edges of the first end section run arcuate, especially convex, in a distance from the tapering, drawn housing section.

2. Nozzle according to claim 1, **characterized in that** the guiding element (12) is carried by the feed tube (7), that the guiding element runs out at its second end (14, 14a, 14b) on the feed tube (7), and **in that** the distance to the second end (14, 14a, 14b) of the guiding element (12) amounts to at least 20% of the length of the feed tube (7), which is arranged inside the housing (1).

3. Nozzle according to one of claims 1 to 2, **characterized in that** adjacent to the second end (14, 14a, 14b) of the guiding element (12) a transitional section (27) is arranged, which forms a constriction (30) with the guiding element (12), and a cylinder section (28) extending along the feed tube (7) is opposite to the guiding element (12) and adjacent to the transitional section (27).

4. Nozzle according to one of the preceding claims, **characterized in that** the first end (13a) of the guiding element (12a) is arranged in the plane in which the outlet opening (9) extends.

5. Nozzle according to one of the preceding claims, **characterized in that** the second dimension (17) of the cross-section (15a, 15b) of the guiding element (12, 12a, 12b) decreases with increasing distance from the longitudinal axis (5).

6. Nozzle according to one of the preceding claims, **characterized in that** the guiding element (12) extends in a first end section (25) adjacent to its first end (13) in the first dimension (16) up to an edge (18), which runs parallel to the inner wall of the housing (1).

7. Nozzle according to one of the preceding claims, **characterized in that** the feed tube (7) extends coaxially with respect to the longitudinal axis (5).

8. Nozzle according to one of the preceding claims, **characterized in that** the feed tube (7) is formed as a bore hole in the guiding element (12, 12a, 12b).

9. Nozzle according to one of the preceding claims, **characterized in that** the feed tube (7) is attached in the guiding element (12, 12a, 12b).

10. Nozzle according to one of the preceding claims, **characterized in that** the guiding element (12, 12a, 12b) is reversibly fixable with the feed tube (7) to a cover (6), which overlaps the housing (1) at its second end (4).

11. Nozzle according to one of the preceding claims, **characterized in that** it has a buffer container (19) for core flow liquid into which the inlet opening (11) of the feed tube (7) discharges.

12. Nozzle according to one of the preceding claims, **characterized in that** it has a buffer container (22) for sheath flow liquid, which is connected to the inlet (23) for sheath flow liquid.

13. Nozzle according to one of the preceding claims, **characterized in that** the edges of the first end section run in a tapering, cone shaped drawn housing section.

14. Nozzle according to claim 13, **characterized in that** the guiding element (12) in the second dimension (17) extends up to at maximum 50 % with respect to the extension in the first dimension (16).

15. Nozzle according to one of the preceding claims, **characterized in that** the cross section of the guiding element (12) in the second dimension (17) decreases along the first dimension (16) from the longitudinal axis (5) of the housing (1) to its edge (18).

16. Method for producing a liquid flow having a particle-containing core flow surrounded by a sheath flow by introducing a particle-containing core flow liquid into an inlet opening (11) for core flow liquid and introducing a sheath flow liquid into an inlet opening (23) for sheath flow liquid of a nozzle according to one of the preceding claims.

17. Method according to claim 16, **characterized in that** the particles have a cross-section, which is smaller in a first dimension (16) that is perpendicular to the longitudinal axis (5) of the housing (1) of the nozzle than in a second dimension (17) that is perpendicular to the longitudinal axis (5) and to the first dimension (16).

18. Method according to one of claims 16 to 17, **characterized in that** at least one property of the particles contained in the core flow is detected in the liquid flow exiting the nozzle and the particles are separated into at least two fractions depending on the detected property, or are treated differently depending on the detected property.

19. Method according to one of claims 16 to 18 for the production of a preparation of particles, which have a cross-section with a first dimension and a second dimension, wherein the particles in the first dimension are smaller than in the second dimension, **characterized in that** the particles are non-human mammalian sperms, the detected property is the presence of the X chromosome or Y chromosome within the sperm, and by the irradiating the sperm in dependence on the detected property or deflection of the sperm in dependence on the detected property into at least two different fractions.

## Revendications

1. Buse pour cytomètre en flux comprenant un logement (1), qui présente une paroi interne symétrique en rotation autour de son axe longitudinal (5) et dont la section transversale interne rétrécit le long de son axe longitudinal (5) depuis sa seconde extrémité (4) vers une sortie (2) au niveau de sa première extrémité (3) et dans lequel est prévu un tube d'arrivée (7) pour courant de liquide primaire, tube de section transversale interne circulaire ou allongée, qui présente une ouverture d'entrée (11) et qui émerge dans une ouverture de sortie (9) prévue au niveau de sa première extrémité, à une certaine distance de la sortie (2), où une entrée (23) est prévue pour un courant liquide d'enveloppe au niveau du logement (1), comprenant un élément de guidage (12) qui s'étend le long de l'axe longitudinal (5), dans une plus grande mesure le long d'une première dimension (16) perpendiculaire à l'axe longitudinal (5) jusqu'à une distance de la paroi interne du logement (1) que par rapport à une seconde dimension (17) perpendiculaire à l'axe longitudinal (5) et à la première dimension (16), où l'élément de guidage (12) s'étend depuis sa première extrémité (13), située dans le plan de la première extrémité (8) du tube d'arrivée (7) ou bien décalée d'une certaine distance par rapport à la première extrémité (8) du tube d'arrivée, jusqu'à sa seconde extrémité (14, 14a, 14b), tube prévu à une certaine distance de la seconde extrémité (4) de la buse (1), **caractérisé en ce que** l'élément de guidage (12) présente une première section d'extrémité (25) au niveau de sa première extrémité (13), section d'extrémité prévue dans une section du logement (1) allant rétrécissant et dont les tranches s'étendent à une certaine distance de la paroi interne du logement (1), et que l'élément de guidage (12) présente une seconde section d'extrémité (26) opposée à la première extrémité (13) et jouxtant celle-ci, seconde section d'extrémité qui rétrécit, dans sa première dimension, en direction de la seconde extrémité (14, 14a, 14b) de l'élément de guidage (12) et opposée à la première extrémité (13), où l'élément de guidage (12) présente son allongement maximal (29) dans la première dimension (16), entre la première section d'extrémité et la seconde section d'extrémité (25, 26), et l'élément de guidage (12) présente une épaisseur qui s'étend dans la seconde dimension (17) de sa section transversale, épaisseur plus petite que son allongement dans la première dimension (16), où les tranches de la première section d'extrémité suivent un parcours en arc-de-cercle, en particulier convexe, à une certaine distance de la section de logement allant rétrécissant.

2. Buse selon la revendication 1, **caractérisée en ce que** l'élément de guidage (12) est supporté par le tube d'arrivée (7), que l'élément de guidage émerge au niveau de sa seconde extrémité (14, 14a, 14b) sur le tube d'arrivée (7) et que la distance de la seconde extrémité (14, 14a, 14b) de l'élément de guidage (12) correspond au moins à 20% de la longueur du tube d'arrivée (7), tube qui est prévu à l'intérieur du logement (1).

3. Buse selon l'une quelconque des revendications 1 ou 2, **caractérisée en ce qu'**une section de transition (27) est prévue limitrophe de la seconde extrémité (14, 14a, 14b) de l'élément de guidage (12), section de transition formant avec l'élément de guidage (12) un rétrécissement (30) et jouxte une section cylindrique (28), en face de l'élément de guidage (12) et jouxtant la section de transition (27), section cylindrique s'étendant le long du tube d'arrivée (7).

4. Buse selon l'une des revendications précédentes, **caractérisée en ce que** la première extrémité (13a) de l'élément de guidage (12a) est prévue dans le plan dans lequel s'étend l'ouverture de sortie (9).

5. Buse selon l'une des revendications précédentes, **caractérisée en ce que** la seconde dimension (17) de la section transversale (15a, 15b) de l'élément de guidage (12, 12a, 12b) rétrécit en s'éloignant de l'axe longitudinal (5).

6. Buse selon l'une des revendications précédentes, **caractérisée en ce que** l'élément de guidage (12) s'étend dans une première section d'extrémité (25) jouxtant sa première extrémité (13) dans la première dimension (16) jusqu'à une tranche (18) s'étendant parallèlement à la paroi interne du logement (1).

7. Buse selon l'une des revendications précédentes, **caractérisée en ce que** le tube d'arrivée (7) s'étend coaxialement à l'axe longitudinal (5).

8. Buse selon l'une des revendications précédentes, **caractérisée en ce que** le tube d'arrivée (7) dans l'élément de guidage (12, 12a, 12b) est en forme d'alésage.

9. Buse selon l'une des revendications précédentes, **caractérisée en ce que** le tube d'arrivée (7) est fixé dans l'élément de guidage (12, 12a, 12b).

10. Buse selon l'une des revendications précédentes, **caractérisée en ce que** l'élément de guidage (12, 12a, 12b) peut être fixé de manière réversible au tube d'arrivée (7) au niveau d'un couvercle (6), couvercle recouvrant le logement (1) au niveau de sa seconde extrémité (4).

11. Buse selon l'une des revendications précédentes, **caractérisée en ce qu'**elle présente un contenant tampon (19) pour courant de liquide primaire, contenant dans lequel émerge l'ouverture d'entrée (11) du tube d'arrivée (7).

12. Buse selon l'une des revendications précédentes, **caractérisée en ce qu'**elle présente un contenant tampon (22) pour courant liquide d'enveloppe, contenant raccordé à l'entrée (23) pour courant liquide d'enveloppe.

13. Buse selon l'une des revendications précédentes, **caractérisée en ce que** les tranches de la première section d'extrémité s'étendent dans une section de logement de forme conique et se rétrécissant.

14. Buse selon la revendication 13, **caractérisée en ce que** l'élément de guidage (12) s'étend dans la seconde dimension (17) sur une distance correspondant au plus à 50% à son extension dans la première dimension (16)

15. Buse selon l'une des revendications précédentes, **caractérisée en ce que** la section transversale de l'élément de guidage (12) rétrécit dans la seconde dimension (17) le long de la première dimension (16) depuis l'axe longitudinal (5) du logement (1) jusqu'à sa tranche (18).

16. Procédé de génération d'un courant liquide avec un courant primaire contenant des particules et entouré d'un courant d'enveloppe en laissant pénétrer un courant de liquide primaire contenant des particules dans une ouverture d'entrée (11) pour courant de liquide primaire et en laissant pénétrer un courant liquide d'enveloppe dans une ouverture d'entrée (23) pour courant liquide d'enveloppe d'une buse selon l'une des revendications précédentes.

17. Procédé selon la revendication 16, **caractérisé en ce que** les particules présentent une section transversale qui est plus petite dans une première dimension (16) perpendiculaire à l'axe longitudinal (5) du logement (1) de la buse, que dans une seconde dimension (17) perpendiculaire à l'axe longitudinal (5) et à la première dimension (16).

18. Procédé selon l'une des revendications 16 ou 17, **caractérisé en ce qu'**au moins une propriété des particules contenues dans le courant primaire est détectée dans le courant primaire sortant de la buse et que les particules sont séparées au moins en deux fractions, en fonction de la propriété détectée, ou bien sont traitées séparément en fonction de la propriété détectée.

19. Procédé selon l'une quelconque des revendications 16 à 18 pour la production d'une préparation de particules, qui présentent une section transversale avec une première dimension et une seconde dimension, où les particules sont plus petites dans la première dimension que dans la seconde dimension, procédé **caractérisé en ce que** les particules constituent du sperme de mammifère non humain, que la propriété détectée porte sur la présence du chromosome X ou du chromosome Y dans le sperme, et fait appel à l'irradiation du sperme en fonction de la propriété détectée ou à la déflexion du sperme en au moins deux fractions différentes en fonction de la propriété détectée.
